(19) Europäisches Patentamt
European Patent Office
Office européen des brevets

(11) Veröffentlichungsnummer: **0 370 219 A1**

# (12) EUROPÄISCHE PATENTANMELDUNG

(21) Anmeldenummer: **89118836.9**

(22) Anmeldetag: **11.10.89**

(51) Int. Cl.[5]: **C07C 323/20, C07C 319/14, C07C 317/22, C07C 315/02**

(30) Priorität: **24.10.88 DE 3836149**

(43) Veröffentlichungstag der Anmeldung: **30.05.90 Patentblatt 90/22**

(84) Benannte Vertragsstaaten: **DE FR GB IT**

(71) Anmelder: **BAYER AG**

**D-5090 Leverkusen 1 Bayerwerk(DE)**

(72) Erfinder: **Marhold, Albrecht, Dr.**
**Carl-Duisberg-Strasse 329**
**D-5090 Leverkusen 1(DE)**
Erfinder: **Bielefeldt, Dietmar, Dr.**
**Beuthener Strasse 13**
**D-4030 Ratingen 6(DE)**
Erfinder: **Baasner, Bernd, Dr.**
**Wagner-Strasse 83**
**D-5060 Bergisch Gladbach 2(DE)**
Erfinder: **Schaller, Klaus, Dr.**
**Am Sonnenschein 38**
**D-5600 Wuppertal 1(DE)**

(54) **Neue 4-Trifluormethylmercaptophenole und ihre Herstellung.**

(57) 4-Trifluormethylmercapto-phenole der Formel

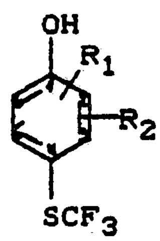

(I)

und 4-Trifluormethylsulfonyl-phenole der Formel (III)

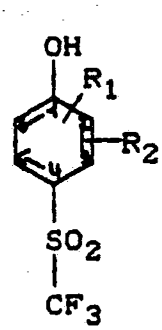

(III),

in denen
$R_1$ und $R_2$ die in der Beschreibung angegebene Bedeutung haben und ein Verfahren zu deren Herstellung

EP 0 370 219 A1

Xerox Copy Centre

aus den entsprechenden Phenolen durch Umsetzung mit Trifluormethylsulfenchlorid und gegebenenfalls anschließender Oxidation.

## Neue 4-Trifluormethylmercapto-phenole und ihre Herstellung

Einige wenige 4-Trifluormethylmercapto-phenole sind bekannt. Beispielsweise kann man gemäß der DE-AS 12 57 784 3-Methyl-4-trifluormethylmercaptophenol herstellen, indem man einen Kohlensäure- oder Chlorkohlensäureester, dessen Alkoholteil aus einer 3-Methyl-4-trichlormethyl-mercapto-phenol-Gruppe besteht, mit Fluorwasserstoff umsetzt und anschließend alkalisch verseift.

Es wurden nun 4-Trifluormethylmercapto-phenole der Formel (I) gefunden

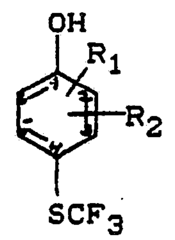

(I),

in der

$R_1$ und $R_2$ unabhängig voneinander jeweils für $C_1$- bis $C_4$-Alkyl, gegebenenfalls substituiertes $C_1$- bis $C_4$-Alkoxy, gegebenenfalls substituiertes $C_1$-bis $C_4$-Alkylthio und/oder Halogen oder

$R_1$ für Wasserstoff und

$R_2$ für $C_2$- bis $C_4$-Alkyl, gegebenenfalls substituiertes $C_1$- bis $C_4$-Alkoxy, gegebenenfalls substituiertes $C_1$-bis $C_4$-Alkylthio oder Fluor

stehen.

Als Substituenten bei gegebenenfalls substituiertem $C_1$-bis $C_4$-Alkoxy und bei gegebenenfalls substituiertem $C_1$-bis $C_4$-Alkylthio kommen bevorzugt Halogene, insbesondere Fluor in Frage.

Soweit $R_1$ und $R_2$ Halogen beteuten kommen Fluor, Chlor, Brom und/oder Iod in Frage.

Vorzugsweise stehen $R_1$ und $R_2$ unabhängig voneinander jeweils für Methyl, Ethyl, Isopropyl, Methoxy, Fluormethoxy, Fluorethoxy, Fluorpropoxy, Fluorbutoxy, Fluormethylthio, Fluorethylthio, Fluorpropylthio, Fluorbutylthio, Fluor, Chlor oder Brom oder

$R_1$ für Wasserstoff und $R_2$ für Ethyl, Isopropyl, Methoxy, Fluormethoxy, Fluorethoxy, Fluorpropoxy, Fluorbutoxy, Fluormethylthio, Fluorethylthio, Fluorpropylthio, Fluorbutylthio, Fluor, Chlor oder Brom,

$C_1$- bis $C_4$-Alkyl steht vorzugsweise für Methyl, Ethyl oder Isopropyl, $C_2$- bis $C_4$-Alkyl vorzugsweise für Ethyl oder Isopropyl. Gegebenenfalls substituiertes $C_1$- bis $C_4$-Alkoxy steht vorzugsweise für Methoxy, Difluormethoxy, Difluorchlormethoxy, Trifluormethoxy, Trifluorethoxy, Tetrafluorethoxy, Trifluorchlorethoxy, Hexafluorpropoxy oder Hexafluorbutoxy.

Gegebenenfalls substituiertes $C_1$- bis $C_4$-Alkylthio steht vorzugsweise für Difluormethylthio, Difluorchlormethylthio, Trifluormethylthio, Trifluorethylthio, Tetrafluorethylthio, Trifluorchlorethylthio, Hexafluorpropylthio oder Hexafluorbutylthio.

Halogen steht vorzugsweise für Fluor, Chlor oder Brom.

$R_1$ und $R_2$ können am aromatischen Ring beliebige Positionen einnehmen, d.h. bei $R_1$ in 2-Position kann $R_2$ in 3-, 5- oder 6-Position vorliegen und bei $R_1$ in 3-Position kann $R_2$ in 2-, 5- und 6-Position vorliegen. Die möglichen Isomeren, bei denen sich $R_1$ in 4- oder 5-Position befindet sind identisch mit den vorgenannten.

Besonders bevorzugte, von der Formel (I) umfaßte Einzelverbindungen enthalten folgende Reste $R_1$ und $R_2$ in den jeweils angegebenen Positionen:

$R_1$ = 3-H, $R_2$ = 1-Ethyl; $R_1$ = 3-H, $R_2$ = 2-Isopropyl; $R_1$ = 3-H, $R_2$ = 2-Methoxy; $R_1$ = $R_2$ = 2,6-Dimethyl; $R_1$ = $R_2$ = 2,3-Dimethyl; $R_1$ = $R_2$ = 3,5-Dimethyl; $R_1$ = $R_2$ = 2,5-Dimethyl; $R_1$ = 2-Methyl, $R_2$ = 5-Chlor; $R_1$ = 2-Methyl, $R_2$ = 3-Chlor; $R_1$ = 2-Ethyl, $R_2$ = 3-Methyl; $R_1$ = 2-Ethyl, $R_2$ = 3-Fluor; $R_1$ = 2-Methyl, $R_2$ = 3-Fluor; $R_1$ = 2-Methyl, $R_2$ = 6-Fluor; $R_1$ = 2-Methyl, $R_2$ = 5-Fluor; $R_1$ = 3-Methyl, $R_2$ = 6-Chlor und $R_1$ = 2-Methyl, $R_2$ = 6-Chlor.

Die vorliegende Erfindung betrifft auch ein Verfahren zur Herstellung von Verbindungen der Formel (I), das dadurch gekennzeichnet ist, daß man ein Phenol der Formel (II)

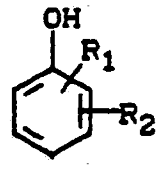

(II),

in der

$R_1$ und $R_2$ die bei Formel (I) angegebene Bedeutung haben

mit Trifluormethylsulfenchlorid umsetzt.

Die erfindungsgemäße Umsetzung zur Herstellung von Verbindungen der Formel (I) kann bei Normaldruck, erhöhtem Druck oder erniedrigtem Druck durchgeführt werden. Vorzugsweise arbeitet man bei Normaldruck.

Die erfindungsgemäße Umsetzung zur Herstellung von Verbindungen der Formel (I) kann beispielsweise bei Temperaturen im Bereich -20 bis +100° C durchgeführt werden. Vorzugsweise arbeitet man bei Temperaturen im Bereich von 0 bis 60° C.

Die Reaktionspartner können im Prinzip in beliebigen Mengenverhältnissen eingesetzt werden. Vorzugsweise setzt man auf 1 Mol Trifluormethylsulfenchlorid 1 bis 10 Mole des jeweiligen Phenols der Formel (II) ein. Besonders bevorzugt setzt man auf 1 Mol Trifluormethylsulfenchlorid 1,05 bis 8 Mole des jeweiligen Phenols der Formel (II) ein. Bei der Verwendung von Phenol im Überschuß kann man im allgemeinen einen quantitativen Verbrauch des Trifluormethylsulfenchlorids und eine Bildung von weniger Nebenprodukten erreichen.

Die erfindungsgemäße Umsetzung zur Herstellung von Verbindungen der Formel (I) kann in Anwesenheit oder Abwesenheit von Katalysatoren durchgeführt werden. Vorzugsweise arbeitet man in Gegenwart von Katalysatoren, weil dann im allgemeinen auch bei relativ niedrigen Temperaturen vorteilhaft gearbeitet werden kann. Als Katalysatoren kommen beispielsweise Lewis-Säuren oder Basen in Frage. Beispiele für Lewis-Säuren sind Eisentrichlorid, Titantetrachlorid, Bortrifluorid, Antimonpentachlorid und Aluminiumtrichlorid. Lewis-Säuren können z.B. in Mengen von 0,01 bis 0,2 Mol pro Mol Trifluormethylsulfenchlorid eingesetzt werden. Beispiele für Basen sind Alkalimetallcarbonate, Triphenylphosphin und tertiäre Stickstoffbasen. Bevorzugt sind tertiäre Amine wie Pyridin, Picolin, Triethylamin, 1,5-Diazabicyclo[4.3.0]-non-5-en und 1,8-Diazabicyclo[5.4.0]-undec-7-en. Basen können z.B. in gleicher oder höherer molarer Menge als Trifluormethylsulfenchlorid eingesetzt werden.

Die erfindungsgemäße Umsetzung zur Herstellung von Verbindungen der Formel (I) kann in Gegenwart oder Abwesenheit von Lösungsmitteln durchgeführt werden. Geeignete Lösungsmittel sind z.B. Ether oder Halogenalkane.

Phenole der Formel (II) sind bekannt und gut zugänglich. Trifluormethylsulfenchlorid ist ebenfalls bekannt und gut zugänglich.

Nach Durchführung der erfindungsgemäßen Umsetzung zur Herstellung von Verbindungen der Formel (I) erhält man häufig Reaktionsgemische, die neben dem gewünschten Produkt auch Bis-trifluormethylmercapto-phenole und gegebenenfalls unumgesetztes Ausgangsphenol enthalten und deshalb aufgearbeitet werden müssen. Die Auftrennung des Reaktionsgemisches ist in den meisten Fällen durch Destillation über eine Kolonne möglich, besonders dann, wenn wenig oder keine Bis-trifluormethylmercaptophenole vorliegen. Teilweise kristallisiert eine Komponente, z.B. überschüssiges Ausgangsphenol oder das gebildete 4-Trifluormethylmercapto-phenol, auch aus den Reaktionsgemisch aus und kann dann durch Filtration abgetrennt werden.

Eine besondere Aufarbeitungsform des Reaktionsgemisches besteht darin, daß man zunächst gegebenenfalls vorhandene Lösungsmittel, Katalysatoren und/oder Hydrochloride von Basen abtrennt, z.B. durch Filtration und/oder einfache Destillation, und anschließend säulenchromatographisch an Kieselgel trennt. Als mobile Phase kann beispielsweise ein Kohlenwasserstoff, insbesondere Toluol, verwendet werden. Im allgemeinen fällt dann als erste Fraktion das Bis-trifluormethylmercapto-substituierte Phenol und als zweite Fraktion das 4-Trifluormethylmercapto-phenol der Formel (I) an. Letzteres kann man in reiner Form gewinnen, indem man die für die säulenchromatographische Trennung eingesetzte mobile Phase, z.B. Toluol, aus der entsprechenden Fraktion entfernt, z.B. durch Destillation.

Die vorliegende Erfindung betrifft weiterhin 4-Trifluormethylsulfonylphenole der Formel (III)

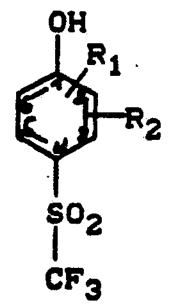

(III),

in der $R_1$ und $R_2$ die bei Formel (I) angegebenene Bedeutung haben. Auch die bevorzugten und besonders bevorzugten Bedeutungen von $R_1$ und $R_2$ in Formel (III) sind wie bei Formel (I) angegeben.

Schließlich betrifft die vorliegende Erfindung auch ein Verfahren zur Herstellung von 4-Fluormethylsulfonyl-phenolen der Formel (III). Dieses Verfahren ist dadurch gekennzeichnet, daß man ein 4-Trifluormethylmercapto-phenol der Formel (I) bei erhöhter Temperatur in Gegenwart einer Säure mit einem aktivierten Sauerstoff enthaltenden Oxidationsmittel oxidiert.

Für diese Oxidation sind beispielsweise Temperaturen im Bereich 50 bis 120 °C geeignet. Vorzugsweise arbeitet man bei 65 bis 100 °C.

Als Säuren kommen z.B. organische Säuren in Frage, wie gegebenenfalls durch Halogen substituierte aliphatische Carbonsäuren mit 1 bis 6 C-Atomen, aber auch Mineralsäuren wie Phosphorsäure oder Schwefelsäure. Bevorzugt sind Essigsäure, Propionsäure, Chloressigsäure und Trifluoressigsäure. Besonders bevorzugt ist Essigsäure.

Als aktivierte Sauerstoffe enthaltende Oxidationsmittel kommen beispielsweise Wasserstoffperoxid, Percarbonsäuren, Caro'sche Säure und deren Salze, Peroxodisulfat und molekularer Sauerstoff in Kombination mit Katalysatoren in Frage. Bevorzugt sind Wasserstoffperoxid, Caro'sche Säure und deren Salze, insbesondere Wasserstoffperoxid, welches beipielsweise in 10 bis 50 gew.-%iger wäßriger Lösung eingesetzt werden kann. Das jeweils eingesetzte Oxidationsmittel kann gegebenenfalls in Wasser und/oder einem organischen Lösungsmittel gelöst sein. Die eingesetzten aktiven Sauerstoff enthaltenden Oxidationsmittel müssen nicht unbedingt in der eingesetzten Form mit der Verbindung der Formel (I) reagieren, sondern sie können auch vor dieser Reaktion ganz oder teilweise in andere aktivierten Sauerstoff enthaltende Oxidationsmittel umgewandelt werden. Beispeilsweise kann aus Wasserstoffperoxid und Schwefelsäure Caro'sche Säure oder aus Wasserstoffperoxid und Essigsäure Peressigsäure entstehen. Ebenso kann man das jeweils gewünschte aktivierten Sauerstoff enthaltende Oxidationsmittel auch erst in situ entstehen lassen, z.B. Caro'sche Säure aus Wasserstoffperoxid und Schwefelsäure.

Das Oxidationsmittel wird vorzugsweise in der stöchiometrisch erforderlichen Menge oder in einem Überschuß von bis zu 100 Mol-% eingesetzt.

Das erfindungsgemäße Verfahren zur Herstellung von 4-Trifluormethylsulfonyl-phenolen der Formel (III) kann in Anwesenheit oder Abwesenheit von Lösungsmitteln durchgeführt werden. Als Lösungsmittel sind Beispielsweise Ether, wie Dioxan oder Diglyme geeignet. Beim Arbeiten in Gegenwart organischer Säuren, insbesondere Essigsäure, kann die Säure auch als Lösungsmittel fungieren.

Die Aufarbeitung des Reaktionsgemisches kann beispielsweise so erfolgen, daß man eventuell vorhandenes überschüssiges Oxidationsmittel zerstört und dann das 4-Trifluormethylsulfonyl-phenol der Formel (III) durch Eingießen in Wasser ausfällt oder durch Abdestillieren von Lösungsmittel und/oder Säure zur Kristallisation bringt und abschließend jeweils filtriert.

Es ist ausgeprochen überraschend, daß es erfindungsgemäß gelingt, 4-Trifluormethylsulfonyl-phenole mit guten Ausbeuten aus den entsprechenden Mercapto-phenolen zu erhalten, denn es sind eine ganze Reihe von Verfahren bekannt, bei denen aus Phenolen durch Oxidation mit Wasserstoffperoxid oder Persäuren die entsprechenden Chinone entstehen.

Die erfindungsgemäßen Verbindungen der Formeln (I) und (III) sind wertvolle Zwischenprodukte zur Herstellung von Produkten, die eine besonders gute Wirkung gegen pflanzenschädigende Pilze haben. Beispielsweise kann man die Verbindungen der Formeln (I) oder (III) mit im aromatischen Kern halogenierten Acetophenonen zu Verbindungen des Typs

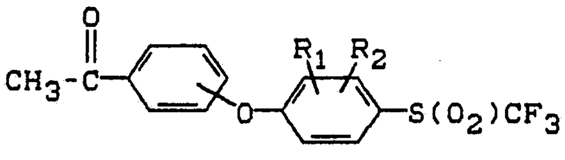

umsetzen, diese an der $CH_3$-Gruppe halogenieren, und dann durch Umsetzung mit einem Thioharnstoffderivat des Typs

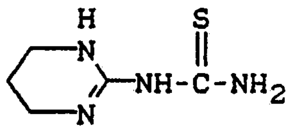

zu Verbindungen des Typs

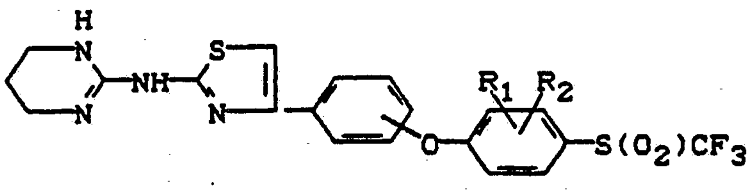

umsetzen, die eine gute Wirkung gegen pflanzenschädigende Pilze haben. Die letztgenannten Verbindungen sind Gegenstand einer anderen eignen Patentanmeldung.

Beispiele:

Beispiele 1 bis 12:

Allgemeine Arbeitsvorschrift:

In 800 ml Di-tert.-butylether wurden 1,64 Mol Phenol der Formel (II) vorgelegt, 1,8 Mol Pyridin zugegeben und bei 20 °C 1,65 bis 1,70 Mol Trifluormethylsulfenchlorid eingeleitet. Dann wurde unter Rühren 4 Stunden auf 50 °C erwärmt. Danach wurde Stickstoff durch das Reaktionsgemisch geblasen (Gasableitung über einen mit wäßrigem Ammoniak gefüllten Wäscher). Danach wurde Pyridinhydrochlorid durch Filtration abgetrennt, leichtflüchtige Bestandteile (im wesentlichen Di-tert.-butylether) unter vermindertem Druck abdestilliert und so rohes 4-Trifluormethylmercapto-phenol erhalten. Die Reinigung erfolgte durch Feindestillation oder durch Chromatographie, wobei die chromatographische Reinigung wie folgt durchgeführt wurde:

Eine Säule (lichte Weite 45 mm) wurde mit einer Aufschlämmung aus Kieselgel in Toluol bis zu einer Höhe von 40 cm gefüllt. Das rohe 4-Trifluormethylmercapto-phenol wurde in wenig Toluol gelöst in die Säule eingetragen. Danach wurde mit Toluol chromatographiert. Aus der jeweiligen 2. Fraktion wurde das Toluol unter vermindertem Druck abdestilliert und so die 4-Trifluormethylmercaptophenole in reiner Form gewonnen.

Die im einzelnen durchgeführten Beispiele sind in Tabelle 1 zusammengefaßt.

**Tabelle 1**

| Beispiel Nr. | Einsatzprodukt 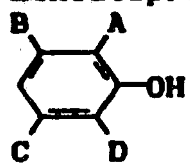 | gereinigtes Endprodukt 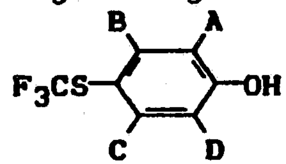 | physikalische Daten des gereinigten Reaktionsprodukt (n.b. = nicht bestimmt) Siedepunkt °C/mbar | $n_D^{20}$ | Schmelzpunkt °C |
|---|---|---|---|---|---|
| 1 | A = $C_2H_5$; B = C = D = Wasserstoff | | 97-98/10 | 1.5030 | n.b. |
| 2 | A = $CH(CH_3)_2$; B = C = D = Wasserstoff | | 92-94/10 | 1.4955 | n.b. |
| 3 | A = $OCH_3$; B = C = D = Wasserstoff | | 94-96/12 | 1.5070 | n.b. |
| 4 | A = D = $CH_3$; B = C = Wasserstoff | | 122-124/22 | n.b. | 38-40 |
| 5 | A = B = $CH_3$; C = D = Wasserstoff | | 120-123/16 | 1.5174 | n.b. |
| 6 | B = C = $CH_3$; A = D = Wasserstoff | | 127-130/16 | n.b. | 68 |
| 7 | A = $CH_3$; B = Cl; C = D = Wasserstoff | | 125-128/16 | n.b. | 48-50*) |
| 8 | A = $CH_3$; C = Cl; B = D = Wasserstoff | | 107-108/10 | 1.5280 | n.b. *) |
| 9 | A = C = $CH_3$; B = D = Wasserstoff | | 104-106/10 | 1.5090 | n.b. |
| 10 | A = Cl; C=$CH_3$; B = D = Wasserstoff | | 125 /14 | 1.5380 | n.b. |
| 11 | A = $C(CH_3)_3$; B = C = D = Wasserstoff | | 110-112/10 | 1.4945 | n.b. |
| 12 | A = C = D = Wasserstoff; B = F | | 72- 73/10 | 1.5045 | n.b. |

*) In diesen Beispielen wurde das Produkt durch Chromatographie gereinigt, sonst wurden die Produkte durch Feindestillation gereinigt.

n.b. nicht bestimmt

Beispiel 13:

4-Trifluormethylmercapto-2,3-dimethylphenol durch $TiCl_4$-Katalyse.

In 500 ml Dichlormethan wurden 100 g 2,3-Dimethylphenol vorgelegt und 10 ml Titantetrachlorid zugegeben. Dann leitete man 50 g Trifluormethylsulfenchlorid bei 20 °C ein und rührte 3 Stunden nach. Danach wurden 100 ml Wasser eingerührt, die Phasen getrennt und die organische Phase destilliert. Nach einem Vorlauf, bestehend aus Dimethylphenol und Produkt, destillierten bei 85-88 °C/6mbar 68 g Produkt.

Beispiele 14 bis 17:

Allgemeine Arbeitsvorschrift:

In 80 ml Essigsäure wurden 0,08 Mol eines 4-Trifluormethylmercapto-phenols der Formel (I) vorgelegt, 35 g 35 %iges Wasserstoffperoxid zugetropft und das Gemisch für 3 Std. auf 90 °C erwärmt. Nach dem Abkühlen wurde das Gemisch in 200 ml Wasser eingerührt und das ausgefallene Produkt filtriert.

Die im einzelnen durchgeführten Beispiele sind in Tabelle 2 zusammengefaßt.

Tabelle 2

| Beispiel Nr. | Einsatzprodukt 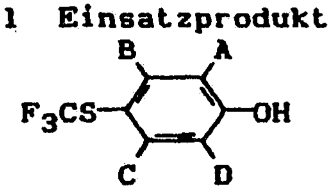 | Reaktionsprodukt 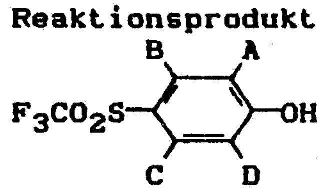 | Schmelzpunkt [°C] | Ausbeute [% d.Th.] |
|---|---|---|---|---|
| 14 | A = $OCH_3$; B = C = D = Wasserstoff | | 121 | 83 |
| 15 | A = $CH_3$; C = Cl; B = D = Wasserstoff | | 111 | 91 |
| 16 | A = B = $CH_3$; C = D = Wasserstoff | | 83 | 74 |
| 17 | A = D = $CH_3$; B = C = Wasserstoff | | 151 | 94 |
| 18 | B, D = $CH_3$, A,C = H | | 72°C | 34 |
| 19 | A = Isopropyl, B, C, D = H | | 69-70°C | 78 |

Ansprüche

1. 4-Trifluormethylmercapto-phenole der Formel (I)

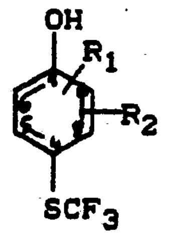

(I),

in der
$R_1$ und $R_2$ unabhängig voneinander jeweils für $C_1$-bis $C_4$-Alkyl, gegebenenfalls substituiertes $C_1$- bis $C_4$-Alkoxy, gegebenenfalls substituiertes $C_1$-bis $C_4$-Alkylthio und/oder Halogen oder
$R_1$ für Wasserstoff und
$R_2$ für $C_2$- bis $C_4$-Alkyl, gegebenenfalls substituiertes $C_1$- bis $C_4$-Alkoxy, gegebenenfalls substituiertes $C_1$- bis $C_4$-Alkylthio oder Fluor
stehen.

2. 4-Trifluormethylmercapto-phenole gemäß Anspruch 1, dadurch gekennzeichnet, daß
$R_1$ und $R_2$ unabhängig voneinander jeweils für Methyl, Ethyl, Isopropyl, Methoxy, Fluormethoxy, Fluorethoxy, Fluorpropoxy, Fluorbutoxy, Fluormethylthio, Fluorethylthio, Fluorpropylthio, Fluorbutylthio, Fluor, Chlor oder brom steht.

3. Verfahren zur Herstellung von 4-Trifluormethylmercapto-phenolen der Formel (I)

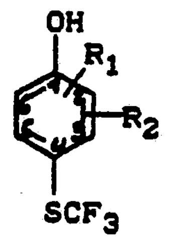

(I),

in der
$R_1$ und $R_2$ unabhängig voneinander jeweils für $C_1$-bis $C_4$-Alkyl, gegebenenfalls substituiertes $C_1$- bis $C_4$-Alkoxy, gegebenenfalls substituiertes $C_1$-bis $C_4$-Alkylthio und/oder Halogen oder
$R_1$ für Wasserstoff und
$R_2$ für $C_2$- bis $C_4$-Alkyl, gegebenenfalls substituiertes $C_1$- bis $C_4$-Alkoxy, gegebenenfalls substituiertes $C_1$- bis $C_4$-Alkylthio oder Fluor stehen, dadurch gekennzeichnet, daß man ein Phenol der Formel (II)

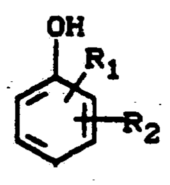

(II),

in der
$R_1$ und $R_2$ die bei Formel (I) angegebene Bedeutung haben
mit Trifluormethylsulfenchlorid umsetzt.

4. Verfahren nach Anspruch 3, dadurch gekennzeichnet, daß man bei Temperaturen im Bereich -20 bis +100 °C arbeitet und man auf 1 Mol Trifluormethylsulfenchlorid 1 bis 10 Mole eines Phenols der Formel (II) einsetzt.

5. Verfahren nach Ansprüchen 3 und 4, dadurch gekennzeichnet, daß man in Gegenwart von Katalysatoren arbeitet.

6. Verfahren nach Ansprüchen 1 bis 5, dadurch gekennzeichnet, daß man in Gegenwart von Lewis-Säuren arbeitet.

7. Verfahren nach Ansprüchen 1 bis 6, dadurch gekennzeichnet, daß man in Gegenwart von Basen arbeitet.

8. 4-Trifluormethylsulfonyl-phenole der Formel (III)

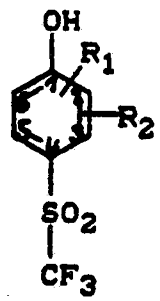

(III),

in der

$R_1$ und $R_2$ unabhängig voneinander jeweils für $C_1$-bis $C_4$-Alkyl, gegebenenfalls substituiertes $C_1$- bis $C_4$-Alkoxy, gegebenenfalls substituiertes $C_1$-bis $C_4$-Alkylthio und/oder Halogen oder

$R_1$ für Wasserstoff und

$R_2$ für $C_2$- bis $C_4$-Alkyl, gegebenenfalls substituiertes $C_1$- bis $C_4$-Alkoxy, gegebenenfalls substituiertes $C_1$-bis $C_4$-Alkylthio oder Fluor

stehen.

9. Verfahren zur Herstellung von 4-Trifluormethylsulfonyl-phenolen der Formel (III)

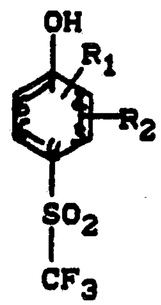

(III),

in der

$R_1$ und $R_2$ unabhängig voneinander jeweils für $C_1$-bis $C_4$-Alkyl, gegebenenfalls substituiertes $C_1$- bis $C_4$-Alkoxy, gegebenenfalls substituiertes $C_1$-bis $C_4$-Alkylthio und/oder Halogen oder

$R_1$ für Wasserstoff und

$R_2$ für $C_2$- bis $C_4$-Alkyl, gegebenenfalls substituiertes $C_1$- bis $C_4$-Alkoxy, gegebenenfalls substituiertes $C_1$-bis $C_4$-Alkylthio oder Fluor

stehen,

dadurch gekennzeichnet, daß man ein 4-Trifluormethylmercapto-phenol der Formel (I)

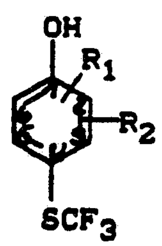

(I),

in der $R_1$ und $R_2$ die gleiche Bedeutung haben wie in Formel (III), bei erhöhter Temperatur in Gegenwart einer Säure mit einem aktivierten Sauerstoff enthaltenden Oxidationsmittel oxidiert.

10. Verfahren nach Anspruch 9, dadurch gekennzeichnet, daß man bei 50 bis 120 °C, in Gegenwart einer gegebenenfalls durch Halogen substituierten aliphatischen Carbonsäure mit 1 bis 6 C-Atomen und mit Wasserstoffperoxid, Percarbonsäuren, Caro'scher Säure oder deren Salze, Peroxodisulfaten oder molekularem Sauerstoff in Kombination mit Katalysatoren als Oxidationsmittel arbeitet.

Europäisches Patentamt

# EUROPÄISCHER TEILRECHERCHENBERICHT,

der nach Regel 45 des Europäischen Patentübereinkommens für das weitere Verfahren als europäischer Recherchenbericht gilt

Nummer der Anmeldung

EP 89 11 8836

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 175 188 (NIHON TOKUSHU NOYAKU SEIZO)<br>* Beispiel VIII-4 *<br>-- | 1-2 | C 07 C 323/20<br>C 07 C 319/14<br>C 07 C 317/22<br>C 07 C 315/02 |
| A | DE-B-1 210 881 (BAYER)<br>----- | | |

RECHERCHIERTE SACHGEBIETE (Int. Cl.4)

C 07 C 323/00
C 07 C 315/00
C 07 C 317/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 2
Unvollständig recherchierte Patentansprüche: 1,3-10
Nicht recherchierte Patentansprüche:
Grund für die Beschränkung der Recherche:

Da in den Patentansprüchen das ungenugend beschreibende Satzteil "gegebenfalls substituierte" ohne nähere Angabe des Substituenten verwendet ist, ist eine vollständige Recherche unmöglich.

| Recherchenort | Abschlußdatum der Recherche | Prüfer |
|---|---|---|
| Den Haag | 22-01-1990 | VAN GEYT |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument